(19) Europäisches Patentamt
European Patent Office
Office européen des brevets

(11) Numéro de publication: **0 053 979**
**B1**

# (12) FASCICULE DE BREVET EUROPEEN

(45) Date de publication du fascicule du brevet: **10.07.85**

(51) Int. Cl.⁴: **C 07 C 35/06,** C 07 C 49/242, C 07 C 49/255, C 07 C 69/732, C 07 C 121/48, C 11 B 9/00, A 61 K 7/46

(21) Numéro de dépôt: **81401920.4**

(22) Date de dépôt: **03.12.81**

(54) **Nouveaux dérivés du cyclopentanol, leur préparation, leur application comme agents parfumants, les compositions les renfermant et les nouveaux intermédiaires obtenus.**

(30) Priorité: **05.12.80 FR 8025869**

(43) Date de publication de la demande: **16.06.82 Bulletin 82/24**

(45) Mention de la délivrance du brevet: **10.07.85 Bulletin 85/28**

(84) Etats contractants désignés: **AT BE CH DE GB IT LI LU NL SE**

(56) Documents cités:
**JOURNAL OF ORGANIC CHEMISTRY, vol. 44, no. 4, 1979, American Chemical Society, EASTON (US), J.J. EISCH et al.: "Stereochemical factors in the carbomagnesiation of unsaturated alcohols", pages 587-593**
**CHEMICAL ABSTRACTS, vol. 90, no. 5, 20 janvier 1979, ref.no. 38592r, page 458, COLUMBUS OHIO (US)**
**CHEMICAL ABSTRACTS, vol. 94, no. 11, 16 mars 1981, ref. 83674n, pages 683,684, COLUMBUS OHIO (US)**
**CHEMICAL ABSTRACTS, vol. 89, no. 13, 25 septembre 1978, ref. 108252k, page 806, COLUMBUS OHIO (US)**

(73) Titulaire: **ROUSSEL-UCLAF, 35, boulevard des Invalides, F-75007 Paris (FR)**

(72) Inventeur: **Martel, Jacques F., 15, rue Douvillez, F-93140 Bondy (FR)**
Inventeur: **Buendia, Jean F., 3 bis Impasse Emilie, F-94170 Le Perreux-sur-Marne (FR)**
Inventeur: **Nezot, François F., 4, allée de Bretagne, F-94320 Thiais (FR)**

(74) Mandataire: **Vieillefosse, Jean-Claude et al, ROUSSEL-UCLAF Boîte postale no. 9 102, route de Noisy, F-93230 Romainville (FR)**

**EP 0 053 979 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés du cyclopentanol, leur procédé de préparation, leur application comme agents parfumants, les compositions les renfermant et les nouveaux intermédiaires obtenus.

L'invention a pour objet les composés de formule (I):

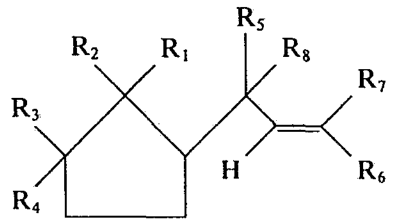

(I)

dans laquelle $R_1$ et $R_2$, identiques, réprésentent un atome d'hydrogène ou un radical alkyle renfermant de 1 à 4 atomes de carbone, $R_3$ représente un radical hydroxyle, un radical $Oalc_1$, $alc_1$ représentant un radical alkyle renfermant de 1 à 8 atomes de carbone, ou un radical Oacyle, acyle représentant le reste d'un acide organique renfermant de 1 à 18 atomes de carbone, $R_4$ représente un atome d'hydrogène ou un radical alkyle renfermant de 1 à 8 atomes de carbone, $R_5$ et $R_8$, identiques ou différents représentent un atome d'hydrogène, un radical alkyle renfermant de 1 à 8 atomes de carbone, un radical alkényle ou alkynyle renfermant de 2 à 8 atomes de carbone, $R_6$ et $R_7$ identiques ou différents représentent un atome d'hydrogène, un radical alkyle renfermant de 1 à 8 atomes de carbone, un radical alkényle ou alkynyle renfermant de 2 à 8 atomes de carbone, un radical $Co_2alc_2$, $alc_2$représentant un radical alkyle renfermant de 1 à 8 atomes de carbone ou un radical $-C{\equiv}N$ ou $CO-CH_3$, à l'exception du allyl cyclopentanol (produit décrit dans J. Org. Chem. Vol. 44 n°4 1979 p. 587 à 593).

Lorsque $R_1$ ou $R_2$ représente un radical alkyle, il s'agit de préférence du radical méthyle, éthyle ou propyle. Lorsque $R_3$ représente un radical $Oalc_1$, on entend de préférence par $alc_1$ un radical alkyle renfermant de 1 à 4 atomes de carbone comme par exemple le radical méthyle, éthyle, n-propyle, isopropyle ou n-butyle,. Lorsque $R_3$ représente un radical Oacyl, on entend de préférence par acyle le reste d'un acide aliphatique ou cycloaliphatique saturé ou insaturé et notamment le reste d'un acide alcanoïque tel que par exemple l'acide acétique, propionique, butyrique ou isobutyrique ou undécylique, le reste d'un acide hydroxyalcanoïque tel que par exemple l'acide hydroxyacétique, le reste d'un acide cycloalcoyl carboxylique ou (cycloalcoyle) alcanoïque tel que par exemple l'acide cyclopropyl, cyclopentyl- ou cyclohexylcarboxylique, cyclopentyl- ou cyclohexyl-acétique ou propionique, le reste d'un acide benzoïque ou d'un acide phenylalcanoïque tel que l'acide phényle acétique ou phényle propionique ou le reste d'un amino acide tel que l'acide diéthylamino acétique ou l'acide aspartique, ou le reste de l'acide formique.

Lorsque $R_4$, $R_5$, $R_6$, $R_7$ ou $R_8$ représente un radical alkyle, il s'agit de préférence d'un radical alkyle renfermant de 1 à 6 atomes de carbone comme, par exemple, le radical méthyle, éthyle, n-propyle, isopropyle ou butyle, pentyle ou hexyle.

Lorsque $R_5$, $R_6$, $R_7$ ou $R_8$ représente un radical alkényle, il s'agit de préférence du radical vinyle, allyle, 2-méthyl allyle ou isobutényle.

Lorsque $R_5$, $R_6$, $R_7$ ou $R_8$ représente un radical alkynyle, il s'agit de préférence du radical éthynyle, 1-propynyle, 2-propynyle, 2-butynyle.

Lorsque $R_6$ ou $R_7$ représente un radical $CO_2$ $alc_2$, $alc_2$ représente de préférence un radical méthyle, éthyle, propyle, isopropyle ou butyle.

L'invention a particulièrement pour objet les composés de formule (I) pour lesquels $R_1$ et $R_2$ représentent un atome d'hydrogène, ainsi que ceux pour lesquelle $R_1$ et $R_2$ représentent un radical méthyle.

Parmi les composés de l'invention, on peut citer plus particulièrement les composés pour lesquels $R_4$ représente un atome d'hydrogène et ceux pour lesquels $R_4$ représente un radical méthyle, ainsi que ceux pour lesquels $R_3$ représente un groupement OH ou $OCOCH_3$ et ceux pour lesquels $R_5$ et $R_8$ représentent un atome d'hydrogène.

L'invention a naturellement tout particulièrement pour objet les composés dont la préparation est donnée plus loin dans la partie expérimentale. Parmi ceux-ci, on peut citer tout spécialement ceux dont les odeurs sont mentionées à l'exemple 20.

Les produits de formule (I) présentent d'intéressantes propriétés organoleptiques qui permettent de les utiliser notamment comme agent parfumants.

Les produits de formule (I) présentent en effet une odeur agréable, certains d'entre eux présentent par exemple une odeur florale, fleurie, fruitée ou boisée pouvant rappeler la rose, le muguet, la bergamote, le concombre, l'odeur du fouin ou du bois sec, l'essence de cèdre ou celle de vétiver.

L'invention a donc pour objet, à titre d'agents parfumants, les produits de formule I, tels que définis précédemment, et notamment parmi ceux-di, les produits préférés mentionnés ci-dessus.

En raison de leurs intéressantes propriétés olfactives, les produits de formule (I) peuvent être utilisés comme agents odorants en parfumerie pour préparer des compositions odorantes qui peuvent servir elles-mêmes de bases à des parfums.

L'invention a donc pour objet les compositions parfumantes, caractérisées en ce qu'elles renferment au moins un agent parfumant tel que défini précédemment.

Les produits de formule (I) peuvent également être utilisés pour la préparation des articles d'hygiène comme par les savons, les talcs, les shampooings, les dentifrices, les sels de bain, les bains moussants, les huiles pour le bain ou les déodorants, pour la préparation des produits cosmétiques comme par exemple les crèmes, les laits démaquillants, les lotions, les fards, les rouges à lèvres et les vernis à ongles.

Les produits de formule (I) peuvent être utilisés pour la préparation de produits detergents, comme par exemple les lessives, ou pour la préparation de produis d'entretien comme les cires, ou enfin pour la préparation des insecticides.

Les composés de formule (I) peuvent apporter une note olfactive à des produits dépourvus d'odeur; ils peuvent également réhausser, exalter ou modifier l'odeur de compositions ayant elles-mêmes une odeur donnée. De plus, comme tout produit présentant une odeur agréable, ils peuvent être utilisés pour masquer l'odeur désagréable d'un produit. Naturellement, les parfums, produits d'hygiène, cosmétiques, produits détergents et produits d'entretien sont réalisés selon les techniques usuelles dans les industries concernées. Ces techniques sont largement décrites dans la littérature spécialisée et n'ont pas à donner lieu ici à des développements particuliers.

Il va de soi que l'invention s'étend aux compositions renfermant, outre les produits de formule (I), les véhicules support, modificateurs, fixateurs, conservateurs, stabilisateurs et autres ingrédients comme les supports, solvants, dispersants et émulsifiants couramment utilisés dans les industries concernées.

Lorsqu'il s'agit de produits utilisés en parfumerie, on peut ajouter aux produits de formule (I) d'autres produits bien connus des parfumeurs, qu'il s'agisse de produits naturels, comme l'essence de vétiver, l'essence de cèdre, l'essence de bergamote, l'essence d'aiguilles de pin, l'essence de citron, l'essence de jasmin ou de mandarine, ou qu'il s'agisse de produits synthétiques, comme les aldéhydes utilisés couramment en parfumerie comme l'hydroxycitronellal, les cétones comme l'$\alpha$-ionone, les composés phénoliques comme l'eugénol des alcools comme le géraniol, les lactones comme la coumarine.

Les quantités de formule (I) à utiliser varient fortement en fonction de la nature de produit choisi, de l'usage que l'on veut en faire, de l'intensité de l'odeur que l'on recherche, ainsi, naturellement, que de la nature et de la composition des autres ingrédients que l'on ajoute au produit de formule (I).

On peut utiliser par exemple de 0,1 à 2% en poids de produits de formule (I) dans le cas de détergents.

Dans le cas de parfums, on peut utiliser par exemple de 0,1 à 10% en poids de produit de formule (I). Lorsqu'il s'agit d'utiliser les produits de formule (I) comme base de parfums, on peut utiliser jusqu'à 20% en poids de produits de formule (I).

L'invention a également pour objet un procédé de préparation des composés de formule (I), caractérisé en ce que l'on soumet, selon la réaction de Wittig, un composé de formule (II)

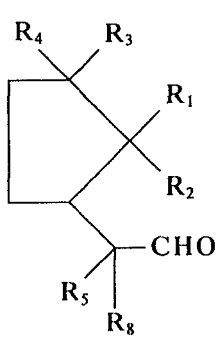

(II)

dans laquelle $R_1$, $R_2$, $R_3$, $R_4$, $R_5$ et $R_8$ conservent la même signification que précédemment, sous forme d'aldéhyde ou sous forme d'hémiacétal correspondant, à l'action d'un composé de formule (III):

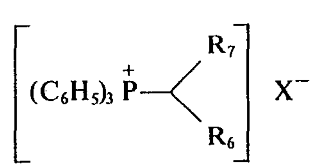

(III)

dans laquelle $X^-$ représente un anion halogènure, pour obtenir le composé de formule (I) correspondant:

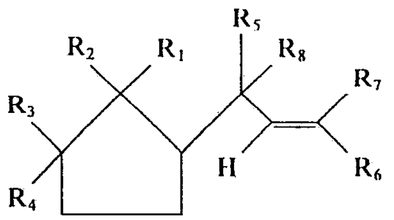

(I)

que l'on soumet, si désiré, dans le cas où $R_3$ représente un groupement hydroxyle, à l'action d'un agent d'éthérification ou d'estérification.

Dans un mode de réalisation préféré du procédé de l'invention,

- X représente un atome de chlore, de brome ou d'iode;
- la réaction de Wittig a lieu au sein d'un solvant choisi dans le groupe constitué par le dimethylformamide, le diméthylsulfoxyde, le tétrahydrofurane, l'éther éthylique, l'éther monoéthylique du diéthylèneglycol, l'éther diéthylique du diéthylèneglycol ou le benzène en présence d'une base forte choisie de préférence dans le groupe constitué par les hydrures alcalins, les amidures alcalins, les alcoolates alcalins, et le butylithium;
- la réaction d'éthérification du composé de formule (I) pour lequel $R_3$ représente un groupement OH est réalisée selon les procédés classifiques, par exemple en forman le dérivé sodé de formule:

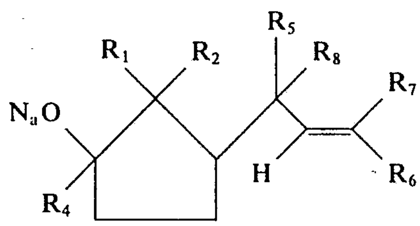

à partir du composé de formule (I), et en faisant réagir ledit composé sur un halogénure d'alkyle, $alc_1$Hal dans lequel $alc_1$ représente un radical alkyle renferment de 1 à 8 atomes de carbone et Hal représente un atome d'halogène;
- la réaction d'estérification des composés de formule (I) lorsque $R_3$ représente un radical hydroxyle est réalisée en faisant réagir le composé de formule (I) avec un dérivé fonctionnel d'un acide, par exemple, un chlorure ou un anhydride d'acide, mais peut également être réalisée en faisant réagir le composé de formule (I) avec un acide en présence de dicyclohexylcarbodiimide ou de diisopropylcarbodiimide.

L'invention comporte une variante évidente pour l'homme de métier du procédé défini ci-dessus, variante qui consiste à soumettre un composé de formule (II) à l'action d'un composé de formule:

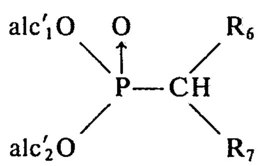

dans laquelle $alc'_1$ et $alc'_2$ représentent un radical alkyle renfermant de 1 à 8 atomes de carbone. Cette variante est particulièrement adaptée pour préparer les composés de formule (I) pour lesquels $R_6$ et $R_7$ ne représentent pas un radical alkyle.

L'invention a également pour objet une variante du procédé général décrit précédemment, caractérisée en ce que l'on soumet un composé de formule (II)

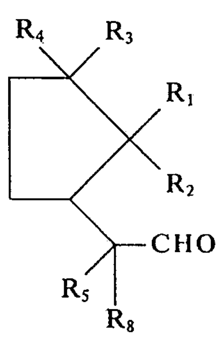

(II)

dans laquelle $R_1$, $R_2$, $R_3$, $R_4$, $R_5$ et $R_8$ conservent la même signification que précédemment, sous forme d'aldéhyde ou sous forme d'hémiacétal correspondant, à l'action d'un composé de formule (IV):

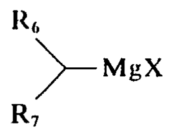

(IV)

dans laquelle $R_6$ et $R_7$ conservent leur signification précédente et X représente un atome d'halogène, pour obtenir le composé de formule (V)

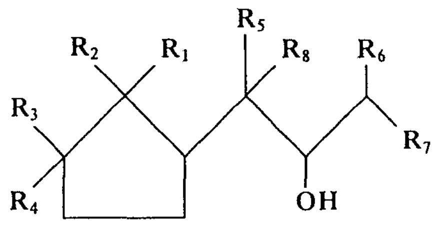

(V)

que l'on soumet à l'action d'un agent de déshydration, pour obtenir le composé de formule (I) correspondant. Dans un mode de réalisation préféré, la déshydration est réalisée par chauffage en milieu acide.

Les composés de formule (II) utilisés comme produits de départ du procédé de l'invention sont des produits nouveaux. L'invention a donc pour objet ces produits, notamment ceux dont la préparation est donnée plus loin dans la partie expérimentale, à titre de produits chimiques nouveaux, et, plus particulièrement à titre de produits intermédiaires nécessaires à la mise en oeuvre du procédé de l'invention, à l'exception du 3-méthoxy $\alpha,\alpha$, 3-triméthyl cyclopentyl acétaldéhyde (produit décrit dans Chemical Abstracts Vol. 94 n° 11 (1981) 83.674n).

Les composés de formule (II) dans lesquels $R_3$ représente un groupement hydroxyle peuvent être préparés en soumettant un composé de formule (VI)

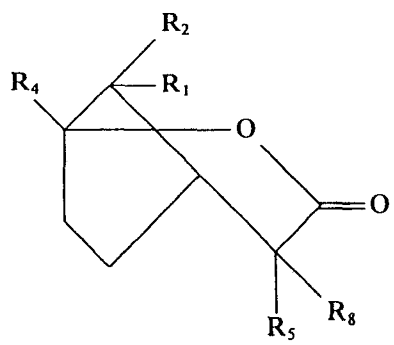

(VI)

à l'action d'un agent d'hydrogénation, par exemple à l'action de l'hydrure de diisobutylaluminium, en opérant à basse température, par exemple entre −40°C et −60°C.

Si on le souhaite, on peut soumettre le composé de formule (II) dans lequel $R_3$ représente un groupement hydroxyle à l'action d'un agent d'éthérification ou d'estérification. On peut réaliser l'éthérification ou l'estérification selon les procédés indiqués plus haut.

Les composés de formule (VI) utilisés pour préparer les produits de formule (II), sont des produits connus qui peuvent être préparés selon le procédé indiqué par J. MEINWALD, Am. Sos. 82, 5235 (1960) ou par RONALD et al, Am. Soc. 81, 925 (1959).

Les produits de formule (V) mis en oeuvre lors de la variante du procédé de l'invention sont des produits nouveaux; l'invention a donc pour objet ces produits à titre de produits chimiques nouveaux, et notamment à titre d'intermédiaires pour préparer les produits de formule (I).

Les exemples suivants illustrent l'invention sans toutefois la limiter.

Exemple 1

3-(2-propényl) cyclopentanol

On ajoute à −60°C en heure trente minutes une solution de 5 g de terbutylate de potassium dans 80 $cm^3$ de diméthylformamide, dans une solution renfermant 5 g de 3-hydroxycyclopentyl

acétaldéhyde, 15 g de bromure de triphényl méthyl phosphonium et 100 $cm^3$ de diméthylformamide. On agite la suspension obtenue pendant 2 heures à −60°C, laisse remonter la température à +20°C, et verse dans une solution d'acide chlorhydrique 2N. On extrait à l'éther isopropylique, sèche la phase organique et amène à sec. On chromatographie le résidu sur silice en éluant par le mélange cyclohexane, acétate d'éthyle 7—3. On obtient 1,7 g de produit recherché.

RMN $CDCl_3$ ppm:
1,6 H de l'OH,
3,7 H du carbone porteur de l'hydroxyle,
5,4 à 6,2 H du carbone en 2 du radical propényle,
4,75 à 5,2 H du carbone en 3 du radical propényle.

Exemple 2

3-(3-méthyl 2-buténynl) cyclopentanol

En opérant comme à l'exemple 1, à partir de 10 g de 3-hydroxy cyclopentyl acétaldehyde et de 37 g d'iodure de triphenyl 1-méthyl éthyl phosphonium, on obtient 8 g d'un produit que l'on chromatographie sur silice en éluant par le mélange cyclohexane acétate d'éthyle 7—3. On obtient ainsi 2 g du produit recherché.

RMN $CDCl_3$ ppm:
2 H de l'OH,
4,2—4,25—4,3 H du carbone porteur de l'hydroxyle,
1,6—1,7 H des méthyles du radical 3-méthyl 2-buténynle,
5—5,2—5,4 H du carbone en 2 du radical 3-méthyl-2-buténynle.

Exemple 3

3-(2-buténynl) cyclopentanol

On ajoute à 20°C en 30 minutes, une solution renfermant 7 g de terbutylate de potassium dans 15 $cm^3$ de diméthylformamide, dans une solution renfermant 5 g de 3-hydroxy cyclopentyl acétaldéhyde, 20 g de bromure de triphényl éthyl phosphonium et 20 $cm^3$ de diméthyl formamide anhydre. On agite le mélange réactionnel 17 heures à 20°C puis le verse dans une solution d'acide chlorhydrique 2N. On extrait à l'éther isopropylique, lave à l'eau la phase organique, sèche et amène à sec. On obtient ainsi 2 g du produit recherché, après chromatographie sur silice en éluant par le mélange cyclohexane acétate d'éthyle (8—2).

RMN $CDCl_3$ ppm:
4,25 H du carbone porteur de OH,
5,4 H des carbones en 2 et 3 du radical buténynle,
1,58—1,65 H du carbone en 4 du radical buténynle.

Exemple 4

3-(2-penténynl) cyclopentanol

En opérant comme à l'exemple 3, à partir de 5 g de 3-hydroxy cyclopentyl acétaldéhyde et de 20 g de bromure de triphényl propyl phosphonium, on obtient après chromatographie sur silice en éluant par le mélange cyclohexane-acétate d'éthyle (7—3) 1,5 g du produit recherché.

RMN $CDCl_3$ ppm:
4,22 H du carbone porteur de OH,
5,3 H des carbones en 2 et 3 du radical penténynle,
0,83—0,95—1,07 H du carbone en 5 du radical penténynle.

Exemple 5

3-(2-hexényl) cyclopentanol

En opérant comme à l'exemple 4, à partir de 5 g du 3-hydroxy cyclopentyl acétaldéhyde et de 20 g de bromure de triphenyl butyl phosphonium, on obtient après chromatographie sur silice en éluant par le mélange cyclohexane acétate d'éthyle (7—3) 1,4 g du produit recherché.

RMN $CDCl_3$ ppm:
4,2 H du carbone porteur de OH,
5,3 H éthyléniques,
0,78—0,9—1,2 H de carbone en 6 de l'hexényle.

Exemple 6

1-acétyloxy 3-(2-butène) cyclopentane

On ajoute à 0°C une solution renfermant 2 $cm^3$ de chlorure d'acétyle et 5 $cm^3$ de benzène, dans une solution renfermant 1 g du produit préparé à l'exemple 3, 20 $cm^3$ de benzène anhydre et 2 $cm^3$ de pyridine. On agite à 20°C pendant 17 heures. On verse la suspension dans l'acide chlorhydrique 2N, agite, décante et extrait par du benzène. On lave à l'eau la solution benzénique, sèche et amène à sec. On chromatographie le résidu sur silice en éluant par le mélange cyclohexane acétate d'éthyle (9—1). On obtient ainsi 1,4 g du produit recherché.

RMN $CDCl_3$ ppm:
5,13 H du carbone porteur de l'acétyloxy,
2,03 H du méthyle de l'acétyloxy,
5,4 H des carbones en 2 et 3 du radical butène,
1,6—1,7 H du carbone en 4 du radical butène.

Exemple 7

(1R,S) 1,2,2-triméthyl 3-(2-propényl) cyclopentanol

On ajoute en une heure trente minutes une solution renfermant 4 g de terbutylate de potassium dans 15 $cm^3$ de diméthylformamide, dans une solution renfermant 5 g de 1,8,8-triméthyl 2-oxabicyclo (3,2,1) octan-3-ol, 15 g de bromure de triphényl méthyl phosphonium et 20 $cm^3$ de diméthylformamide. On agite le mélange réactionnel pendant deux heures à 20°C puis verse la suspension dans une solution d'acide chlorhydrique 2N et extrait à l'éther isopropylique. On lave à l'eau, sèche et amène à sec. On chromatographie le résidu sur silice en éluant par le mélange cyclohexane acétate d'éthyle (8—2) et obtient le produit attendu.

RMN $CDCl_3$ ppm:
1,2 H du méthyle porté par le même carbone que OH,
0,83 et 0,88 H des méthyles en 2 du cyclopentanol,
5,5 à 6,2 H du carbone en 2 du radical propényle,
4,8 à 5,2 H du carbone en 3 du radical propényle.

Exemple 8

(1R,S) 1,2,2-triméthyl 3-(5-méthyl 2,4-hexényl) cyclopentanol

En opérant comme à l'exemple 7, à partir de 16 g de chlorure de 3-méthyl 2-butényle triphényl phosphonium et de 5 g de 1,8,8-triméthyl 2-oxabicyclo (3,2,1) octan-3-ol, on obtient après chromatographie sur silice en éluant par le mélange cyclohexane acétate d'éthyle (8—2), 1,7 g de produit attendu.

RMN $CDCl_3$ ppm:
0,87 H des méthyles en 2,
1,17 H du méthyle en 1 du cyclopentanol,
4,8 à 7,3 H des carbones en 2, 3 et 4 du radical 2,4-hexényle,
1,73—1,8 H des méthyles du radical 5-méthyl 2,4-hexényle.

### Exemple 9

(1R,S) 1,2,2-triméthyl 3-(2-buténye) cyclopentanol

En opérant comme à l'exemple 7, à partir de 5 g de 1,8,8-triméthyl 2-oxabicyclo (3,2,1) octan 3-ol et de 15 g de bromure de triphényl éthyl phosphonium on obtient après purification par chromatographie sur silice en éluant par le mélange cyclohexane acétate d'éthyle (8—2), 4 g du produit recherché.

RMN $CDCl_3$ ppm:
1,17 H de méthyle en 1,
0,85 H des méthyles en 2,
5,4 H des carbones en 2 et 3 du radical buténye,
1,6 à 1,7 H du carbone en 4 du radical buténye.

### Exemple 10

(1R,S) 1,2,2-triméthyl 3-(3-méthyl 2-buténye) cyclopentanol

En opérant comme à l'exemple 7, à partir de 5 g de 1,8,8-triméthyl 2-oxa bicyclo [3,2,1] octan 3-ol et de 15 g d'iodure de triphényl isopropyl phosphonium, on obtient après purification par chromatographie sur silice en éluant par le mélange cyclohexane acétate d'éthyle (8—2), 4,1 g du produit recherché.

RMN $CDCl_3$ ppm:
0,83 et 0,86 H des méthyles en 2,
1,81 H du méthyle en 1,
1,62—1,68 H des méthyles du radical 3-méthyl 2-buténye.

### Exemple 11

(1R,S) 1,2,2-triméthyl 3-(2-pentenyl) cyclopentanol

En opérant comme à l'exemple 7, à partir de 5 g de 1,8,8-triméthyl 2-oxy bicyclo [3,2,1] -octan 3-ol et de 15 g de bromure de triphenyl propyl phosphonium, on obtient après purification par chromatographie sur silice en éluant par le mélange cyclohexane acétate d'éthyle (8—2), 4 g du produit recherché.

RMN $CDCl_3$ ppm:
0,83 et 0,86 H des méthyles en 2,
1,17 H du méthyle en 1,
5,33 H éthyléniques du penténye,
0,83—0,93—1,05 H du carbone en 5 du radical penténye.

### Exemple 12

(1R,S) 1,2,2-triméthyl 3-(2-hexényl) cyclopentanol

En opérant comme à l'exemple 7 à partir de 5 g de 1,8,8-triméthyl 2-oxa-bicyclo [3,2,1] octan 3-ol et de 15 g de bromure de triphényl butyl phosphonium, on obtient après chromatographie sur silice, en éluant par le mélange cyclohexane-acétate d'ethyle (8—2), 4 g du produit recherché.

RMN $CDCl_3$ ppm:
0,87 et 0,9 H des méthyles en 2,
1,2 H du méthyle en 1,
5,5 H des carbones en 2 et 3 de l'hexényl,
0,85—0,95—1,05 H des carbone en 6 de l'hexényle.

Exemple 13

(1 R,S) 1,2,2-triméthyl 3-(3-méthyl 2-butène 4-carboxylate de méthyle) cyclopentanol

On introduit en 15 minutes à 0°C, 8 g de 1-méthoxy carbonyl éthyl phosphonate de 0,0-diéthyle, dans une solution renferment 2 g de méthylate de sodium dans 10 $cm^3$ de méthanol. On ajoute ensuite en une heure trente minutes 5 g de 1,8,8-triméthyl 2-oxa bicyclo [3,2,1] octan-3-ol dans 5 $cm^3$ de benzène. On maintient le mélange réactionnel sous agitation pendant 3 heures à 20°C, le verse dans une solution d'acide chlorhydrique 2N, décante et extrait au benzène. On lave à l'eau, sèche et amène à sec. On chromatographie le résidu sur silice en éluant par le mélange benzène-acétate d'éthyle (8—2). On obtient ainsi 2 g du produit recherché.

RMN $CDCl_3$ ppm:
0,87 H des méthyles en 2,
1,37 H de l'OH,
1,17 H du méthyle en 1,
6,6—6,7—6,8 H du carbone en 2 du radical 3-méthyl 2-butényl,
3,72 H du méthyle ester,
1,83 H du méthyle en 3.

Exemple 14

(1 R,S) 1,2,2-triméthyl 3-/2 (E)-propène 3-carboxylate d'éthyle/cyclopentanol

On chauffe au reflux pendant 3 heures une solution renferment 12 g d'éthoxycarbonyl méthylène triphényl phosphorane, 5 g de 1,8,8-triméthyl 2-oxa bicyclo [3,2,1] octan -3ol et 50 $cm^3$ de benzène anhydre. On distille le benzène, reprend le résidu par de l'éther isopropylique, essore l'insoluble, lave à l'éther isopropylique et amène à sec le filtrat. On chromatographie le résidu sur silice en éluant par le mélange benzène-acétate d'éthyle (8—2). On obtient ainsi 1,5 g du produit recherché.

RMN $CDCl_3$ ppm:
0,85 et 0,87 H des méthyles en 2,
1,18 H du méthyle en 1,
{ 6,7—6,8—6,9 H du carbone en 2 du radical propényle,
{ 6,9—7—7,1,
{ 1,17—1,28—1,4 H de l'ester éthylique,
{ 4—4,1—4,2,
5,7—5,9 H du carbone en 3 du radical propényle.

Exemple 15

Acétate de (1 R,S) 1,2,2-triméthyl 3-(2-hexéne) cyclopentyle

On porte au reflux pendant 3 heures un mélange renferment 2 g de (1 R,S) 1,2,2-triméthyl 3-(2-hexényl) cyclopentanol, 100 $cm^3$ de benzène, 30 $cm^3$ de pyridine, 20 $cm^3$ de chlorure d'acétyle. On refroidit à 20°C et verse la suspension obtenue dans une solution d'acide chlorhydrique 2N. On agite, décante, extrait au benzène, sèche la phase organique et amène à sec. On chromatographie le résidu en éluant par le benzène. On abtient ainsi 1,9 g du produit recherché.

RMN $CDCl_3$ ppm:
0,85—0,93 H des méthyles en 2,
1,38 H du méthyle en 1,
1,98 H de l'acétoxy,
5,33 H des carbones en 2 et 3 de l'hexènyle,
0,92 H du carbone en 6 de l'héxényle.

Exemple 16

Acétate de (1 R,S) 1,2,2-triméthyl 3-(2-butène) cyclopentyle

En opérant comme à l'exemple 15, à partir de 2 g de (1 R,S) 1,2,2-triméthyl 3-(2-butène) cyclopentanol et de 15 $cm^3$ de chlorure d'acétyle, on obtient 1,8 g du produit recherché.

RMN $CDCl_3$ ppm:
{ 0,85 et 0,93 { 0,82 et 0,92 } H des méthyles en 2,
1,38 H du méthyle en 1,
1,98 H de l'acétoxy,
5,3 H des carbones en 2 et 3 du radical butenyle,
1,56—1,65 H du carbone en 4 du radical butenyle.

Exemple 17

Acétate de (1 R,S) 1,2,2-triméthyl 3-(2-propényl) cyclopentyle

En opérant comme à l'exemple 15, à partir de 1,6 g du (1 R,S) 1,2,2-triméthyl 3-(2-propényl) cyclopentanol et de 10 $cm^3$ de chlorure d'acétyle, on obtient 1,3 g du produit recherché.

RMN $CDCl_3$ ppm:
0,83 et 0,93 H des méthyles en 2,
1,38 H du méthyle en 1,
1,98 H de l'acétoxy,
5,5 à 6,25 H du carbone en 2 du radical propényle,
4,8 à 5,2 H du carbone en 3 du radical propényle.

Exemple 18

Acétate de (1 R,S) 1,2,2-triméthyl 3-(3-méthyl 2-buténył) cyclopentyle

En opérant comme à l'exemple 15, à partir de 2 g de (1 R,S) 1,2,2-triméthyl 3-(3-méthyl 2-butényl) cyclopentanol et de 3 $cm^3$ de chlorure d'acétyle, on obtient après chromatographie sur silice en éluant par le mélange cyclohexaneacétate d'éthyle (95—5), 1,5 g du produit recherché.

RMN $CDCl_3$ ppm:
0,83 et 0,92 H des méthyles en 2,
1,37 H du méthyle en 1,
1,98 H de l'acétoxy,
4,9—5,1—5,3 H du carbone en 2 du radical 3-méthyl 2-buténylе,
1,6—1,7 H des méthyles du radical 3-méthyl 2-buténylе.

Exemple 19

Acétate de (1 R,S) 1,2,2-triméthyl 3-(2-penténył) cyclopentyle

En opérant comme à l'exemple 15, à partir de 2 g de (1 R,S) 1,2,2-triméthyl 3-(2-pentényl) cyclopentanol et de 10 $cm^3$ de chlorure d'acétyle, on obtient 1,5 g du produit recherche.

RMN $CDCl_3$ ppm:
0,85 et 0,93 H des méthyles en 2,
1,38 H du méthyle en 1,
1,97 H de l'acétoxy,
5,3 H des carbones en 2 et 3 du radical penténylе,
0,85—0,95—1,05 H du carbone en 5 du radical penténylе.

Les produits utilisés comme produits de départ des exemples 1 à 19 sont préparés de la façon suivante:

Préparation 1

3-hydroxy cyclopentyl acétaldehyde

On ajoute, goutte à goutte, à —60°C, 55 $cm^3$ d'une solution 1,2 M d'hydrure de diisobutylaluminium dans le toluène, à une solution renfermant 8 g de 2-oxa bicyclo [3,2,1] 3-octanone (préparé selon J. Meinvald Am. Soc. 82 5235, 1960) dans le toluène. On maintient le mélange réactionnel à —60°C sous agitation pendant 15 minutes et le verse dans une solution d'acide chlorhydrique N glacé.

On décante la phase toluénique, la sèche et la concentre à sec sous pression réduits sans dépasser 25°C. On obtient 0,8 g de produit. Des eaux-mères extraites au chlorure de méthylène, on obtient 7 g de produit. On réunit les deux fractions (7,8 g), les chromatographie sur silice en éluant au mélange benzène-acétate d'éthyle (1—1) et obtient ainsi 6 g de produit recherché.

RMN $CDCl_3$ ppm:
4,5 H en 3 du cyclopentane,
9,8 H du formyle.

Préparation 2

1,8,8-triméthyl 2-oxa bicyclo [3,2,1] octan 3-ol

On introduit en une heure trente minutes à −60°C, 22 cc d'une solution 1,1 M d'hydrure de diisobutylaluminium dans le toluène dans une solution renfermant 5 g de 1,8,8-triméthyl 2-oxa bicyclo [3,2,1] octane 3-one, préparé selon le procédé décrit par Ronald et al Am. Soc. 81 925 (1959) dans 20 cc de toluène. On agite deux heures à −60°C et ajoute de l'eau goutte à goutte en laissant remonter la température à 0°C. On agite pendant deux heures à 20°C, filtre les sels d'aluminium, lave au chlorure de méthylène. On décante le filtrat, extrait au chlorure de méthylène, sèche et amène à sec. On chromatographie le résidu sur silice en éluant par le mélange benzène-acétate d'éthyle 50—50, pour obtenir 3,5 g de produit attendu.

RMN $CDCl_3$ ppm:
0,85 et 1,1 H des méthyles en 1 et 8,
3,4—3,5 H de l'OH,
5 à 5,4 H du carbone en 3.

Exemple 20

Odeurs dégagées par certains produits de formule I

Exemple 1: odeur de foin indéfinissable
Exemple 2: acétal fleuri du carbinol-muguet
Exemple 3: rosé
Exemple 4: rose et concombre
Exemple 5: gras — concombre
Exemple 6: très joli fleuri pour des roses
Exemple 8: cèdre, boisé sec
Exemple 10: fruité
Exemple 17: note orangée
Exemple 18: bergamote

Exemple 21

Exemple de base de parfums

On a préparé des bases de parfums répondant à la composition suivante.

On a préparé des formules de Composition »Opoponax« à partir des ingrédients ci-après (parties en poids):

| | |
|---|---|
| — Bergamote | 310 |
| — Néroli de synthèse | 20 |
| — Patchouli déferrisé | 10 |
| — Rose essence | 10 |
| — Vétyvérol | 60 |
| — Santalol | 125 |
| — Résinoide Castoréum | 40 |
| — Coumarine | 80 |
| — Méthylionone Gamma | 75 |
| — Vanilline | 40 |
| — Résinoide Benjoin | 25 |
| — Musc Kétone | 40 |
| — Musc Ambrette | 65 |
| — Produit de l'exemple 3 | 100 |
| | 1000 |

Exemple 22

Exemple de détergens

On a préparé des poudres de détergents répondant à la composition suivante:

- Poudre de détergents du commerce 5000 parties en poids
- Produit de l'exemple 18 1 partie en poids

Exemple 23

Exemple de savons

On a préparé des savons répondant à la composition suivante:

- Savons du commerce 1000 parties en poids
- Produit de l'exemple 2 5 parties en poids

**Revendications pour les Etats contractants: BE, CH, DE, GB, IT, LI, LU, NL, SE**

1. Les composés de formule (I)

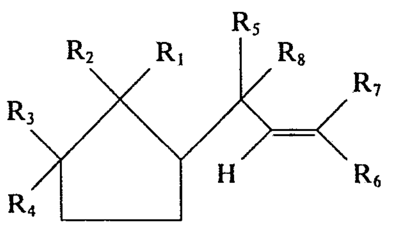

(I)

dans laquelle $R_1$ et $R_2$, identiques, représentent un atome d'hydrogène ou un radical alkyle renfermant de 1 à 4 atomes de carbone, $R_3$ représente un radical hydroxyle, un radical $Oalc_1$, $alc_1$ représentant un radical alkyle renfermant de 1 à 8 atomes de carbone, ou un radical Oacyle, acyle représentant le reste d'un acide organique renfermant de 1 à 18 atomes de carbone, $R_4$ représente un atome d'hydrogène ou un radical alkyle renfermant de 1 à 8 atomes de carbone, $R_5$ et $R_8$, identiques ou différents, représentent un atome d'hydrogène, un radical alkyle renfermant de 1 à 8 atomes de carbone, un radical alkényle ou alkynyle renfermant de 2 à 8 atomes de carbone, $R_6$ et $R_7$, identiques ou différents représentent un atome d'hydrogène, un radical alkyle renfermant de 1 à 8 atomes de carbone, un radical alkényle ou alkynyle renfermant de 2 à 8 atomes de carbone, un radical $Co_2alc_2$, $alc_2$représentant un radical alkyle renfermant de 1 à 8 atomes de carbone, ou un radical $-C{\equiv}N$ ou $CO-CH_3$ à l'exception du 3-allyl cyclopentanol.

2. Les composés de formule (I) tels que définis à la revendication 1, pour lesquels $R_1$ et $R_2$ répresentent un atome d'hydrogène.

3. Les composés de formule (I) tels que définis à la revendication 1, pour lesquels $R_1$ et $R_2$ représentent un radical méthyle.

4. Les composés de formule (I) tels que définis à l'une quelconque des revendications 1 à 3, pour lesquels $R_4$ représente un atome d'hydrogène.

5. Les composés de formule (I) tels que définis à l'une quelconque des revendications 1 à 3, pour lesquels $R_4$ représente un radical méthyle.

6. Les composés de formule (I) tels que définis à l'une quelconque des revendications 1 à 5, pour lesquels $R_3$ représente un groupement OH ou $OCOCH_3$.

7. Les composés de formule (I) tels que définis à l'une quelconque des revendications 1 à 6, pour lesquels $R_5$ et $R_8$ représentent un atome d'hydrogène.

8. A titre d'agents parfumants les composés de formule (I)

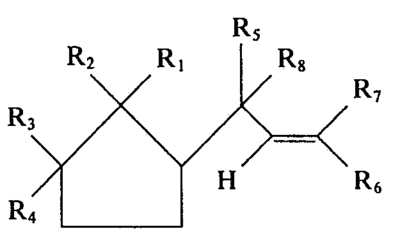

(I)

dans laquelle $R_1$ et $R_2$, identiques, représentent un atome d'hydrogène ou un radical alkyle renfermant de 1 à 4 atomes de carbone, $R_3$ représente un radical hydroxyle, un radical $Oalc_1$, $alc_1$ représentant un radical alkyle renfermant de 1 à 8 atomes de carbone, ou un radical Oacyle, acyle représentant le reste d'un acide organique renfermant de 1 à 18 atomes de carbone, $R_4$ représente un atome d'hydrogène ou un radical alkyle renfermant de 1 à 8 atomes de carbone, $R_5$ et $R_8$, identiques ou différents, représentent un atome d'hydrogène, un radical alkyle renfermant de 1 à 8 atomes de carbone, un radical alkényle ou alkynyle renfermant de 2 à 8 atomes de carbone, $R_6$ et $R_7$, identiques ou différents représentent un atome d'hydrogène, un radical alkyle renfermant de 1 à 8 atomes de carbone, un radical alkényle ou alkynyle renfermant de 2 à 8 atomes de carbone, un radical $Co_2alc_2$, $alc_2$représentant un radical alkyle renfermant de 1 à 8 atomes de carbone, ou un radical $-C{\equiv}N$ ou $CO-CH_3$, tels que définis à l'une quelconque des revendications 1 à 7.

9. Les compositions parfumantes renfermant comme principe actif au moins un agent parfumant tel que défini à la revendication 8.

10. Procédé de préparation des composés de formule (I) tels que définies à l'une quelconque des revendications 1 à 7, caractérisé en ce que l'on soumet selon la réaction de Wittig un composé de formule (II)

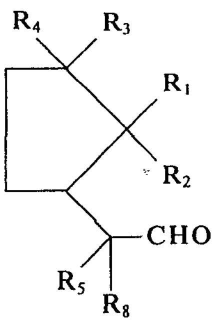

(II)

dans laquelle $R_1$, $R_2$, $R_3$, $R_4$, $R_5$ et $R_8$ conservent la même signification que dans la revendication 1, sous forme d'aldéhyde ou sous forme d'hémiacétal correspondant, à l'action d'un composé de formule (III):

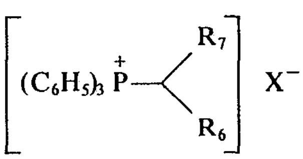

(III)

dans laquelle $X^-$ représente un anion halogénure, pour obtenir le composé de formule (I) correspondant:

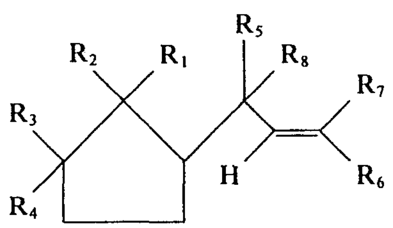

(I)

que l'on soumet, si désiré, dans le cas où $R_3$ représente un groupement hydroxyle, à l'action d'un agent d'éthérification ou d'estérification.

11. Variante du procédé selon la revendication 10, caractérisée en ce que l'on soumet un composé de formule (II)

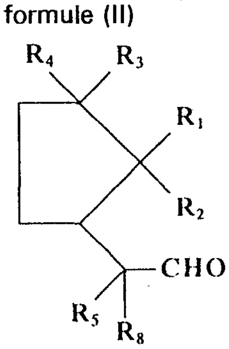

(II)

dans laquelle $R_1$, $R_2$, $R_3$, $R_4$, $R_5$ et $R_8$ conservent la même signification que dans la revendication 1, sous forme d'aldéhyde ou sous forme d'hémiacétal correspondant, à l'action d'un composé de formule (IV):

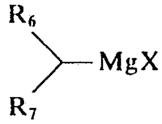

(IV)

dans laquelle $R_6$ et $R_7$ conservent leur signification précédente et X représente un atome d'halogène, pour obtenir le composé de formule (V)

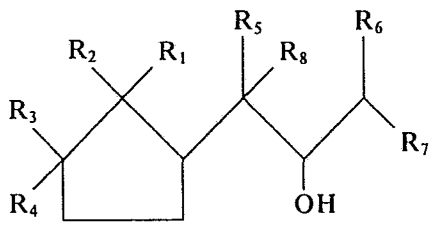

(V)

que l'on soumet à l'action d'un agent de déshydration, pour obtenir le composé de formule (I) correspondant.

12. A titre de produits intermédiaires nouveaux, nécessaires à la mise en oeuvre du procédé selon la revendication 10 ou 11, les composés de formule (II) à l'exception du 3-méthoxy $\alpha,\alpha$, 3-triméthyl cyclopentylacétaldéhyde, leurs hémiacétals et les composés de formule (V), tels que définis à la revendication 10 ou 11.

13. A titre de produits intermédiaires tels que définis à la revendication 12, le 3-hydroxy cyclopentyl acétaldéhyde selon la formule (II) et le 1,8,8-triméthyl 2-oxa bicyclo [3,2,1] octan-3-ol qui est l'hémiacétal d'un composé de formule (II).

**Revendications pour l'Etat contractant: AT**

1. Procédé de préparation des composés de formule (I):

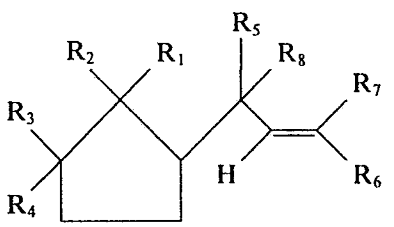

(I)

dans laquelle $R_1$ et $R_2$, identiques, représentent un atome d'hydrogène ou un radical alkyle renfermant de 1 à 4 atomes de carbone, $R_3$ représente un radical hydroxyle, un radical $Oalc_1$, $alc_1$ représentant un radical alkyle renfermant de 1 à 8 atomes de carbone, ou un radical Oacyle, acyle représentant le reste d'un acide organique renfermant de 1 à 18 atomes de carbone, $R_4$ représente un atome d'hydrogène ou un radical alkyle renfermant de 1 à 8 atomes de carbone, $R_5$ et $R_8$, identiques ou différents, représentent un atome d'hydrogène, un radical alkyle renfermant de 1 à 8 atomes de carbone, un radical alkényle ou alkynyle renfermant de 2 à 8 atomes de carbone, $R_6$ et $R_7$, identiques ou différents représentent un atome d'hydrogène, un radical alkyle renfermant de 1 à 8 atomes de carbone, un radical alkényle ou alkynyle renfermant de 2 à 8 atomes de carbone, un radical $Co_2alc_2$, $alc_2$représentant un radical alkyle renfermant de 1 à 8 atomes de carbone, ou un radical $-C{\equiv}N$ ou $CO-CH_3$ à l'exception du 3-allyl cyclopentanol, caractérisé en ce que l'on soumet selon la réaction de Wittig un composé de formule (II):

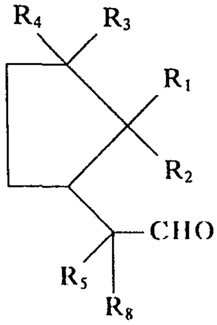

(II)

dans laquelle $R_1$, $R_2$, $R_3$, $R_4$, $R_5$ et $R_8$ conservent la même signification que dans la revendication 1, sous forme d'aldéhyde ou sous forme d'hémiacétal correspondant, à l'action d'un composé de formule (III):

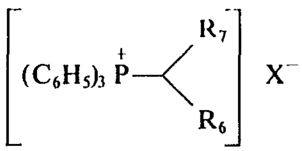

(III)

dans laquelle $X^-$ représente un anion halogénure, pour obtenir le composé de formule (I) correspondant:

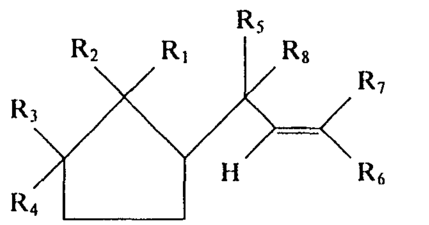

(I)

que l'on soumet, si désiré, dans le cas où $R_3$ représente un groupement hydroxyle, à l'action d'un agent d'éthérification ou d'estérification.

2. Variante du procédé selon la revendication 10, caractérisée en ce que l'on soumet un composé de formule (II)

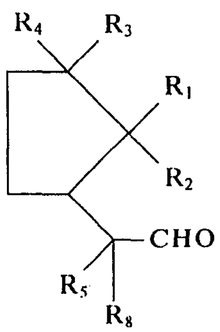

(II)

dans laquelle $R_1$, $R_2$, $R_3$, $R_4$, $R_5$ et $R_8$ conservent la même signification que dans la revendication 1, sous forme d'aldéhyde ou sous forme d'hémiacétal correspondant, à l'action d'un composé de formule (IV):

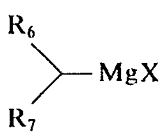

(IV)

dans laquelle $R_6$ et $R_7$ conservent leur signification précédente et X représente un atome d'halogène, pour obtenir le composé de formule (V)

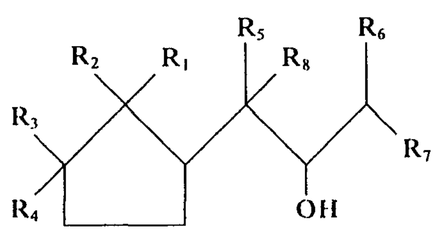

(V)

que l'on soumet à l'action d'un agent de déshydration, pour obtenir le composé de formule (I) correspondant.

**Patentansprüche für die Vertragsstaaten: BE, CH, DE, GB, IT, LI, LU, NL, SE**

1. Verbindungen mit der Formel (I)

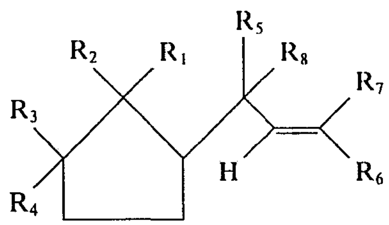

(I)

worin $R_1$ und $R_2$, die gleich sind, ein Wasserstoffatom oder einen Alkylrest mit 1 bis 4 Kohlenstoffatomen bedeuten, $R_3$ einen Hydroxylrest, einen Rest $OAlk_1$, worin $Alk_1$ einen Alkylrest mit 1 bis 8 Kohlenstoffatomen bedeutet, oder einen Rest OAcyl, worin Acyl den Rest einer organischen Säure mit 1 bis 18 Kohlenstoffatomen bedeutet, darstellt, $R_4$ ein Wasserstoffatom oder einen Alkylrest mit 1 bis 8 Kohlenstoffatomen darstellt, $R_5$ und $R_8$, die gleich oder verschieden sind, ein Wasserstoffatom, einen Alkylrest mit 1 bis 8 Kohlenstoffatomen, einen Alkenyl- oder Alkinylrest mit 2 bis 8 Kohlenstoffatomen bedeuten, $R_6$ und $R_7$, die gleich oder verschieden sind, ein Wasserstoffatom, einen Alkylrest mit 1 bis 8 Kohlenstoffatomen, einen Alkenyl- oder Alkinylrest mit 2 bis 8 Kohlenstoffatomen und einen Rest $CO_2Alk_2$, worin $Alk_2$ einen Alkylrest mit 1 bis 8 Kohlenstoffatomen bedeutet oder einen Rest $-C{\equiv}N$ oder $CO-CH_3$ bedeuten, mit der Ausnahme von 3-Allylcyclopentanol.

2. Verbindungen der Formel (I) wie in Anspruch 1 definiert, worin $R_1$ und $R_2$ ein Wasserstoffatom bedeuten.

3. Verbindungen der Formel (I) wie in Anspruch 1 definiert, worin $R_1$ und $R_2$ einen Methylrest bedeuten.

4. Verbindungen der Formel (I) wie in einem der Ansprüche 1 bis 3 definiert, worin $R_4$ ein Wasserstoffatom bedeutet.

5. Verbindungen der Formel (I) wie in einem der Ansprüche 1 bis 3 definiert, worin $R_4$ einen Methylrest bedeutet.

6. Verbindungen der Formel (I) wie in einem der Ansprüche 1 bis 5 definiert, worin $R_3$ eine Gruppe OH oder $OCOCH_3$ bedeutet.

7. Verbindungen der Formel (I) wie in einem der Ansprüche 1 bis 6 definiert, worin $R_5$ und $R_8$ ein Wasserstoffatom bedeuten.

8. Als Duftstoffe die Verbindungen der Formel (I)

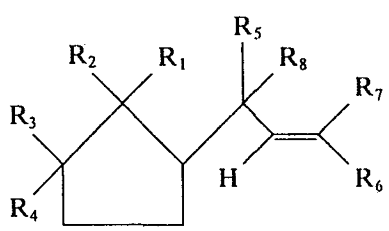

(I)

worin $R_1$ und $R_2$, die gleich sind, ein Wasserstoffatom oder einen Alkylrest mit 1 bis 4 Kohlenstoffatomen bedeuten, $R_3$ einen Hydroxylrest, einen Rest $OAlk_1$, worin $Alk_1$ einen Alkylrest mit 1 bis 8 Kohlenstoffatomen darstellt, oder einen Rest OAcyl, worin Acyl den Rest einer organischen Säure mit 1 bis 18 Kohlenstoffatomen bedeutet, darstellt, $R_4$ ein Wasserstoffatom oder einen Alkylrest mit 1 bis 8 Kohlenstoffatomen bedeutet, $R_5$ und $R_8$, die gleich oder verschieden sind, ein Wasserstoffatom, einen Alkylrest mit 1 bis 8 Kohlenstoffatomen, einen Alkenyl- oder Alkinylrest mit 2 bis 8 Kohlenstoffatomen bedeuten, $R_6$ und $R_7$, die gleich oder verschieden sind, ein Wasserstoffatom, einen Alkylrest mit 1 bis 8 Kohlenstoffatomen, einen Alkenyl- oder Alkinylrest mit 2 bis 8 Kohlenstoffatomen, einen Rest $CO_2Alk_2$, worin $Alk_2$ einen Alkylrest mit 1 bis 8 Kohlenstoffatomen bedeutet, oder einen Rest $-C{\equiv}N$ oder $CO-CH_3$ bedeuten, wie in einem der Ansprüche 1 bis 7 definiert.

9. Duftstoffzusammensetzungen, enthaltend als aktives Prinzip mindestens einen Duftstoff wie in Anspruch 8 definiert.

10. Verfahren zur Herstellung der Verbindungen der Formel (I) wie in einem der Ansprüche 1 bis 7 definiert, dadurch gekennzeichnet, daß man eine Verbindung der Formel (II)

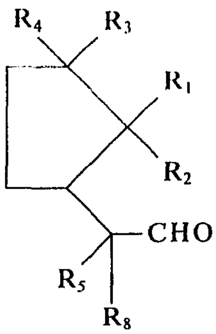

(II)

worin $R_1$, $R_2$, $R_3$, $R_4$, $R_5$ und $R_8$ die gleiche Bedeutung wie in Anspruch 1 beibehalten, in der Form des Aldehyds oder in der Form des entsprechenden Hemiacetals einer Wittig-Reaktion durch Einwirkung einer Verbindung der Formel (III)

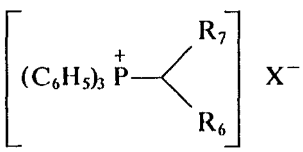

(III)

unterwirft, worin $X^-$ ein Halogenidanion darstellt, zur Erzielung der Verbindung der entsprechenden Formel (I)

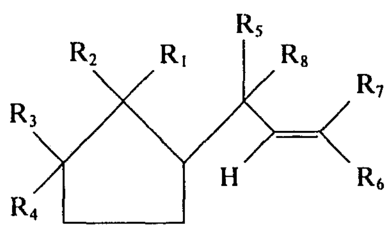

(I)

die man, falls gewünscht, für den Fall daß $R_3$ eine Hydroxylgruppe darstellt, der Einwirkung eines Veretherungs- oder Veresterungsmittels unterwirft.

11. Ausführungsform des Verfahrens nach Anspruch 10, dadurch gekennzeichnet, daß man eine Verbindung der Formel (II)

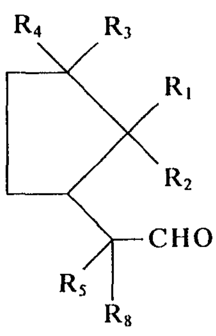

(II)

worin $R_1$, $R_2$, $R_3$, $R_4$, $R_5$ und $R_8$ die gleiche Bedeutung wie in Anspruch 1 beibehalten, in der Form des Aldehyds oder in der Form des entsprechenden Hemiacetals der Einwirkung einer Verbindung der Formel (IV)

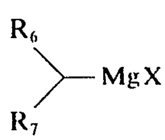

(IV)

unterwirft, worin $R_6$ und $R_7$ ihre vorstehende Bedeutung beibehalten und X ein Halogenatom darstellt, zur Erzielung der Verbindung der Formel (V)

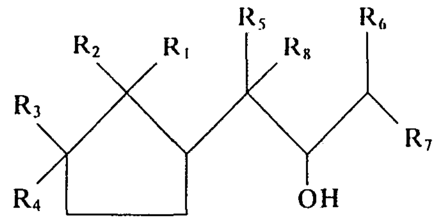

(V)

die man der Einwirkung eines Entwässerungsmittels unterwirft, um die Verbindung der entsprechenden Formel (I) zu erhalten.

12. Als neue Zwischenprodukte, die zur Durchführung des Verfahrens nach Anspruch 10 oder 11 benötigt werden, die Verbindungen der Formel (II), mit Ausnahme von 3-Methoxy-$\alpha$,$\alpha$-3-trimethylcyclopentylacetaldehyd, deren Hemiacetale und die Verbindungen der Formel (V), wie in Anspruch 10 oder 11 definiert.

13. Als Zwischenprodukte, wie in Anspruch 12 definiert, der 3-Hydroxy-cyclopentylacetaldehyd der Formel (II) und das 1,8,8-Trimethyl-2-oxa-bicyclo-[3,2,1]-octan-3-ol, das das Hemiacetal einer Verbindung der Formel (II) ist.

**Patentansprüche für den Vertragsstaat: AT**

1. Verfahren zur Herstellung der Verbindungen der Formel (I)

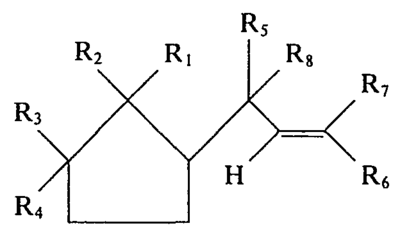

(I)

worin $R_1$ und $R_2$, die gleich sind, ein Wasserstoffatom oder einen Alkylrest mit 1 bis 4 Kohlenstoffatomen bedeuten, $R_3$ einen Hydroxylrest, einen Rest $OAlk_1$, worin $Alk_1$ einen Alkylrest mit 1 bis 8 Kohlenstoffatomen bedeutet oder einen Rest OAcyl, worin Acyl den Rest einer organischen Säure mit 1 bis 18 Kohlenstoffatomen bedeutet, darstellt, $R_4$ ein Wasserstoffatom oder einen Alkylrest mit 1 bis 8 Kohlenstoffatomen bedeutet, $R_5$ und $R_8$, die gleich oder verschieden sind, ein Wasserstoffatom, einen Alkylrest mit 1 bis 8 Kohlenstoffatomen, einen Alkenyl- oder Alkinylrest mit 2 bis 8 Kohlenstoffatomen bedeuten, $R_6$ und $R_7$, die gleich oder verschieden sind, ein Wasserstoffatom, einen Alkylrest mit 1 bis 8 Kohlenstoffatomen, einen Alkenyl- oder Alkinylrest mit 2 bis 8 Kohlenstoffatomen, einen Rest $CO_2Alk_2$, worin $Alk_2$ einen Alkylrest mit 1 bis 8 Kohlenstoffatomen bedeutet, oder einen Rest $-C{\equiv}N$ oder $CO-CH_3$ darstellen, mit der Ausnahme von 3-Allylcyclopentanol, dadurch gekennzeichnet, daß man eine Verbindung der Formel (II)

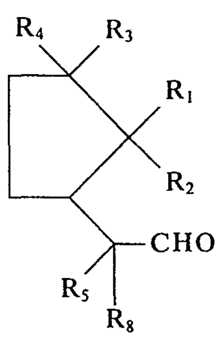

(II)

worin $R_1$, $R_2$, $R_3$, $R_4$, $R_5$ und $R_8$ die gleiche Bedeutung wie in Anspruch 1 beibehalten, in der Form des Aldehyds oder in der Form des entsprechenden Hemiacetals einer Wittig-Reaktion durch Einwirkung einer Verbindung der Formel (III)

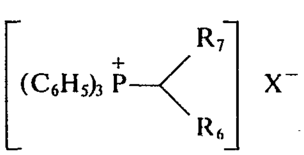

(III)

unterwirft, worin X ein Halogenidanion darstellt, zur Erzielung der Verbindung der entsprechenden Formel (I)

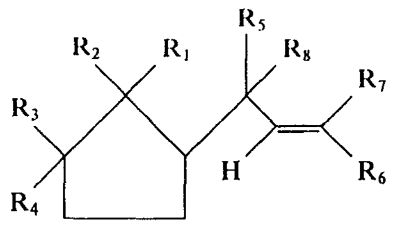

(I)

die man, falls gewünscht, falls $R_3$ eine Hydroxylgruppe darstellt, der Einwirkung eines Veretherungs- oder Veresterungsmittels unterwirft.

2. Ausführungsform des Verfahrens nach Anspruch 10, dadurch gekennzeichnet, daß man eine Verbindung der Formel (II)

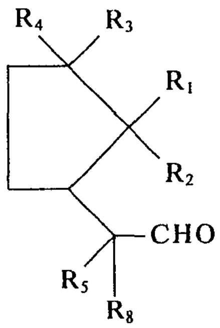

(II)

worin $R_1$, $R_2$, $R_3$, $R_4$, $R_5$ und $R_8$ die gleiche Bedeutung wie in Anspruch 1 beibehalten, in der Form des Aldehyds oder in der Form des entsprechenden Hemiacetals der Einwirkung einer Verbindung der Formel (IV)

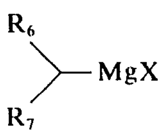

(IV)

unterwirft, worin $R_6$ und $R_7$ ihre vorstehende Bedeutung beibehalten und X ein Halogenatom darstellt, zur Erzielung der Verbindung der Formel (V)

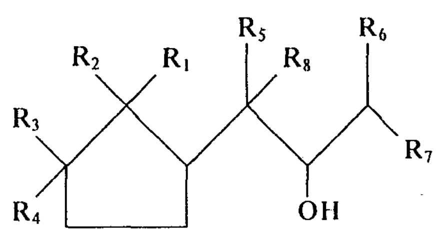

(V)

die man der Einwirkung eines Entwässerungsmittels unterwirft, um die Verbindung der entsprechenden Formel (I) zu erhalten.

**Claims for the contracting States: BE, CH, DE, GB, IT, LI, LU, NL, SE**

1. Compounds with the formula (I)

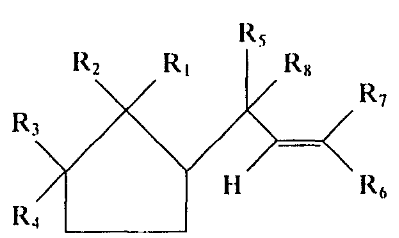

(I)

in which $R_1$ and $R_2$, being identical, each represent a hydrogen atom or an alkyl radical containing from 1 to 4 carbon atoms, $R_3$ represents a hydroxyl radical, an $Oalk_1$ radical, $alk_1$ representing an alkyl radical containing from 1 to 8 carbon atoms, or an Oacyl radical, acyl representing the residue of an organic acid containing from 1 to 18 carbon atoms, $R_4$ represents a hydrogen atom or an alkyl radical containing from 1 to 8 carbon atoms, $R_5$ and $R_8$, identical or different, each represent a hydrogen atom, an alkyl radical containing from 1 to 8 carbon atoms, an alkenyl or alkynyl radical containing from 2 to 8 carbon atoms, $R_6$ and $R_7$, identical or different, each represent a hydrogen atom, an alkyl radical containing from 1 to 8 carbon atoms, an alkenyl or alkynyl radical containing from 2 to 8 carbon atoms, a $CO_2alk_2$ radical, $alk_2$ representing an alkyl radical containing from 1 to 8 carbon atoms, or a $-C{\equiv}N$ or $CO-CH_3$ radical with the exception of 3-allyl cyclopentanol.

2. Compounds with the formula (I) as defined in Claim 1, for which $R_1$ and $R_2$ each represent a hydrogen atom.

3. Compounds with the formula (I) as defined in Claim 1, for which $R_1$ and $R_2$ each represent a methyl radical.

4. Compounds with the formula (I) as defined in any one of the Claims 1 to 3, for which $R_4$ represents a hydrogen atom.

5. Compounds with the formula (I) as defined in any one of the Claims 1 to 3, for which $R_4$ represents a methyl radical.

6. Compounds with the formula (I) as defined in any one of the Claims 1 to 5, for which $R_3$ represents an OH or $OCOCH_3$ group.

7. Compounds with the formula (I) as defined in any one of the Claims 1 to 6, for which $R_5$ and $R_8$ each represent a hydrogen atom.

8. As perfuming agents the compounds with the formula (I)

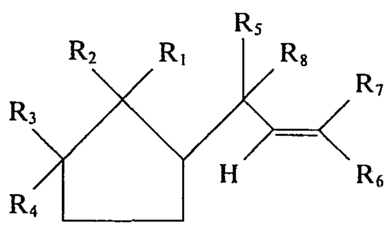

(I)

in which $R_1$ and $R_2$, being identical, each represent a hydrogen atom or an alkyl radical containing from 1 to 4 carbon atoms, $R_3$ represents a hydroxyl radical, an $Oalk_1$ radical, $alk_1$ representing an alkyl radical containing from 1 to 8 carbon atoms, or an Oacyl radical, acyl representing the residue of an organic acid containing from 1 to 18 carbon atoms, $R_4$ represents a hydrogen atom or an alkyl radical containing from 1 to 8 carbon atoms, $R_5$ and $R_8$, identical or different, each represent a hydrogen atom, an alkyl radical containing from 1 to 8 carbon atoms, an alkenyl or alkynyl radical containing from 2 to 8 carbon atoms, $R_6$ and $R_7$, identical or different, each represent a hydrogen atom, an alkyl radical containing from 1 to 8 carbon atoms, an alkenyl or alkynyl radical containing from 2 to 8 carbon atoms, a $CO_2alk_2$ radical, $alk_2$ representing an alkyl radical containing from 1 to 8 carbon atoms, or a $-C{\equiv}N$ or $CO-CH_3$ radical, as defined in any one of the Claims 1 to 7.

9. Perfuming compositions containing as active principle at least one perfuming agent as defined in Claim 8.

10. Preparation process for compounds with the formula (I) as defined in any of the Claims 1 to 7, characterized in that a compound with the formula (II)

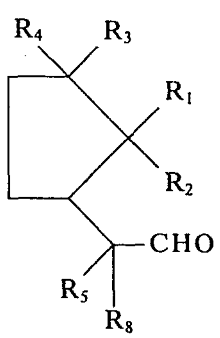

(II)

in which $R_1$, $R_2$, $R_3$, $R_4$, $R_5$ and $R_8$ retain the same significance as in Claim 1, in the form of an aldehyde or in the form of the corresponding hemiacetal, is submitted according to the Wittig reaction, to the action of a compound with the formula (III):

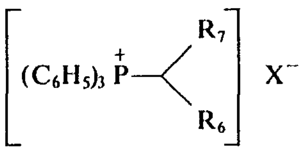

(III)

in which $X^-$ represents a halogenide anion, so as to obtain the corresponding compound with the formula (I):

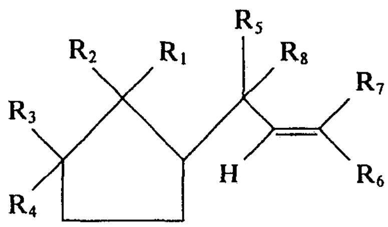

(I)

which is submitted, if desired, in the case when $R_3$ represents a hydroxyl group, to the action of a etherification or esterification agent.

11. A variant of the process according to Claim 10, characterized in that a compound with the formula (II)

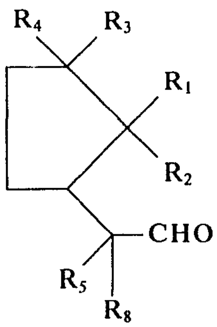

(II)

in which $R_1$, $R_2$, $R_3$, $R_4$, $R_5$ and $R_8$ retain the same significance as in Claim 1, in the form of an aldehyde or in the form of the corresponding hemiacetal, is submitted to the action of a compound with the formula (IV):

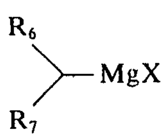

(IV)

in which $R_6$ and $R_7$ retain their previous significance and X represents a halogen atom, so as to obtain the compound with the formula (V)

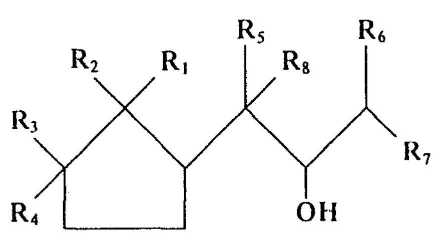

(V)

which is submitted to the action of a dehydration agent, so as to obtain the corresponding compound with the formula (I).

12. As new intermediary products, necessary for carrying out the process according to Claim 10 or 11, compounds with the formula (II) with the exception of 3-methoxy *α,α*, 3-trimethylcyclopentylacetaldehyde, their hemiacetals and compounds with the formula (V), as defined in Claim 10 or 11.

13. As intermediary products as defined in Claim 12, 3-hydroxy cyclopentyl acetaldehyde according to formula (II) and 1,8,8-trimethyl-2-oxabicyclo[3,2,1]octan-3-ol which is the hemiacetal of a compound with the formula (II).

**Claims for the contracting State: AT**

1. Preparation process for compounds with the formula (I)

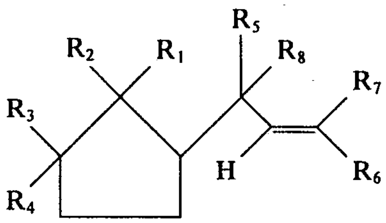

(I)

in which $R_1$ and $R_2$, being identical, each represent a hydrogen atom or an alkyl radical containing from 1 to 4 carbon atoms, $R_3$ represents a hydroxyl radical, an $Oalk_1$ radical, $alk_1$ representing an alkyl radical containing from 1 to 8 carbon atoms, or an Oacyl radical, acyl representing the residue of an organic acid containing from 1 to 18 carbon atoms, $R_4$ represents a hydrogen atom or an alkyl radical containing from 1 to 8 carbon atoms, $R_5$ and $R_8$, identical or different, each represent a hydrogen atom, an alkyl radical containing from 1 to 8 carbon atoms, an alkenyl or alkynyl radical containing from 2 to 8 carbon atoms, $R_6$ and $R_7$, identical or different, each represent a hydrogen atom, and an alkyl radical containing from 1 to 8 carbon atoms, an alkenyl or alkynyl radical containing from 2 to 8 carbon atoms, a $CO_2alk_2$ radical, $alk_2$ representing an alkyl radical containing from 1 to 8 carbon atoms, or a $-C{\equiv}N$ or $CO{-}CH_3$ radical, with the exception of 3-allyl cyclopentanol, characterized in that a compound with the formula (II):

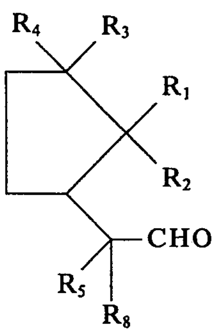

(II)

in which $R_1$, $R_2$, $R_3$, $R_4$, $R_5$ and $R_8$ retain the same significance as in Claim 1, in the form of an aldehyde or in the form of the corresponding hemiacetal, is submitted according to the Wittig reaction, to the action of a compound with the formula (III):

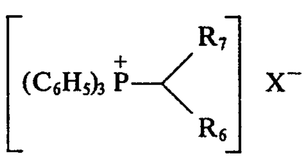

(III)

in which $X^-$ represents a halogenide anion, so as to obtain the corresponding compound with the formula (I):

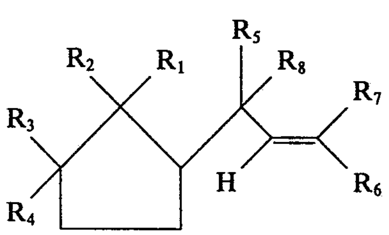

(I)

which is submitted, if desired, in the case when $R_3$ represents a hydroxyl group, to the action of an etherification or esterification agent.

2. A variant of the process according to Claim 1, characterized in that a compound with the formula (II)

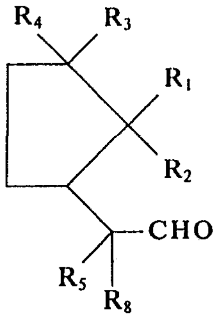

(II)

in which $R_1$, $R_2$, $R_3$, $R_4$, $R_5$ and $R_8$ retain the same significance as in Claim 1, in the form of an aldehyde or in the form of the corresponding hemiacetal, is submitted to the action of a compound with the formula (IV):

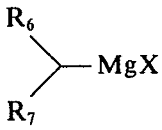

(IV)

in which $R_6$ and $R_7$ each retain their previous significance and X represents a halogen atom, so as to obtain the compound with the formula (V)

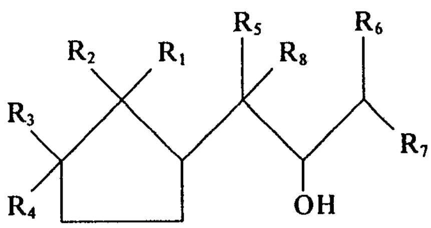

(V)

which is submitted to the action of a dehydration agent, so as to obtain the corresponding compound with the formula (I).